# EUROPEAN PATENT APPLICATION

(11) **EP 2 154 189 A1**
(43) Date of publication of application: **17.02.2010**
(21) Application number: 09167107.3
(22) Date of filing: 03.08.2009
(51) Int. Cl.: C08K 5/107, C07C 69/54

(54) **Phenol composition and thermoplastic polymer composition comprising the composition**

(30) Priority: 12.08.2008 JP 2008207664
(71) Applicant: Sumitomo Chemical Company, Limited, Tokyo 104-8260 (JP)
(72) Inventor: Sato, Natsuko, Osaka (JP); Soma, Ryoji, Osaka (JP)
(74) Representative: Harmsen, Dirk

(57) **Abstract**

Recently, further improved process stability for thermoplastic polymer compositions has been demanded. Phenol compositions are provided comprising a compound represented by the formula (1): 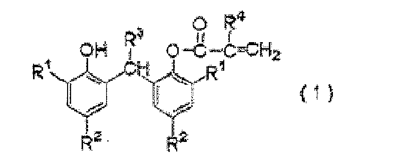 in the formula (1), R¹ and R² are each independently an alkyl group having 1 to 8 carbon atoms, an aryl group having 6 to 18 carbon atoms, or an aralkyl group having 7 to 18 carbon atoms; R³ is an alkyl group having 1 to 3 carbon atoms; and R⁴ is a hydrogen atom or a methyl group, and a compound represented by the formula (2)_{:} 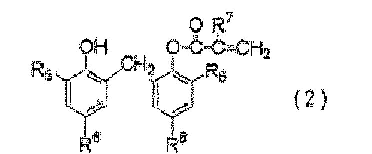 in the formula (2), R⁵ and R⁶ are each independently an alkyl group having 1 to 18 carbon atoms, an aryl group having 6 to 18 carbon atoms, or an aralkyl group having 7 to 18 carbon atoms; and R⁷ is a hydrogen atom or a methyl group, in a weight ratio of 1:99 to 99:1.

## Description

The present application is filed, claiming the Paris Convention priorities of Japanese Patent Application No. 2008-207664 (filed on August 12, 2008), the entire content of which is incorporated herein by reference.

The present invention relates to compositions comprising phenol, thermoplastic polymer compositions comprising the composition, and the like.

Thermoplastic polymers generally have high transparency and good impact resistance, but they have defects of worse process stability, such as deterioration of flowability or generation of a fish eye gel during a molding process.

As thermoplastic polymer compositions having excellent process stability, polybutadiene rubber compositions comprising, for example, 2,4-di-t-amyl-6-[1-(3,5-di-t-amyl-2-hydroxyphenyl)ethyl]phenyl acrylate (hereinafter sometimes referred to as "compound (1-1)") (Japanese Patent Application Laid-Open Publication No. 1-168643 [applied Examples 1 and 2]), and butadiene polymer compositions comprising, for example, 2-t-butyl-6-(3-t-butyl-2-hydroxy-5-methylbenzyl)-4-methylphenyl acrylate (hereinafter sometimes referred to as "compound (2-1)") (Japanese Patent Application Laid-Open Publication No. 62-18445 [Reference Example 1, and Examples 1 to 3 of the Invention]) are proposed.

Recently, further improved process stability for thermoplastic polymer compositions has been demanded.

In order to overcome the defect, the present inventors have devoted themselves to finding compounds to be mixed with a thermoplastic polymer. As a result, they have reached the inventions [1] to [6] as described below.
[1] A phenol composition comprising:
   a compound represented by the formula (1): 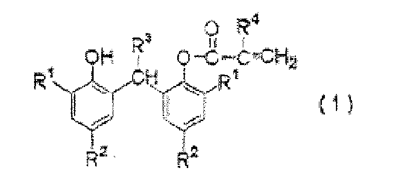
   in the formula (1), R¹ and R² are each independently an alkyl group having 1 to 8 carbon atoms, an aryl group having 6 to 18 carbon atoms, or an aralkyl group having 7 to 18 carbon atoms; R³ is an alkyl group having 1 to 3 carbon atoms; and R⁴ is a hydrogen atom or a methyl group, and a compound represented by the formula (2): 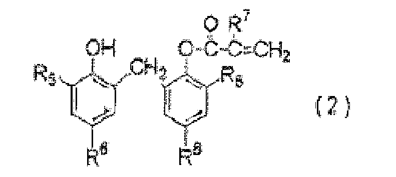
   in the formula (2), R⁵ and R⁶ are each independently an alkyl group having 1 to 18 carbon atoms, an aryl group having 6 to 18 carbon atoms, or an aralkyl group having 7 to 18 carbon atoms; and R⁷ is a hydrogen atom or a methyl group, in a weight ratio of 1:99 to 99:1.
[2] The phenol composition of [1], wherein the compound represented by the formula (1) is
   2,4-di-t-amyl-6-[1-(3,5-di-t-amyl-2-hydroxyphenyl)ethyl]phenyl acrylate, and the compound represented by the formula (2) is 2-t-butyl-6-(3-t-butyl-2-hydroxy-5-methylbenzyl)-4-methylphenyl acrylate.
[3] A thermoplastic polymer composition comprising:
   a thermoplastic polymer, a compound represented by the formula (1): 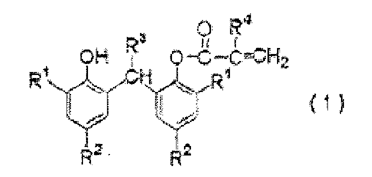
   in the formula (1), R¹ and R² are each independently an alkyl group having 1 to 8 carbon atoms, an aryl group having 6 to 18 carbon atoms, or an aralkyl group having 7 to 18 carbon atoms; R³ is an alkyl group having 1 to 3 carbon atoms; and R⁴ is a hydrogen atom or a methyl group, and a compound represented by the formula (2): 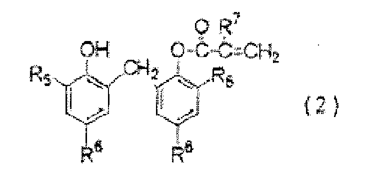
   in the formula (2), R⁵ and R⁶ are each independently an alkyl group having 1 to 18 carbon atoms, an aryl group having 6 to 18 carbon atoms, or an aralkyl group having 7 to 18 carbon atoms; and R⁷ is a hydrogen atom or a methyl group, wherein
   a weight ratio of the compound represented by the formula (1) to the compound represented by the formula (2) in the composition is from 1:99 to 99:1.
[4] The thermoplastic polymer composition of [3], wherein the total contents of the compound represented by the formula (1) and the compound represented by the formula (2) is from 0.01 to 5 parts by weight based on 100 parts by weight of the thermoplastic polymer.
[5] A process for producing a thermoplastic polymer composition comprising the step of:
   mixing a thermoplastic polymer with a compound represented by the formula (1): 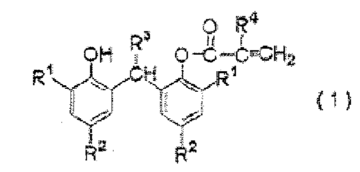
   in the formula (1), R¹ and R² are each independently an alkyl group having 1 to 8 carbon atoms, an aryl group having 6 to 18 carbon atoms, or an aralkyl group having 7 to 18 carbon atoms; R³ is an alkyl group having 1 to 3 carbon atoms; and R⁴ is a hydrogen atom or a methyl group and a compound represented by the formula (2): 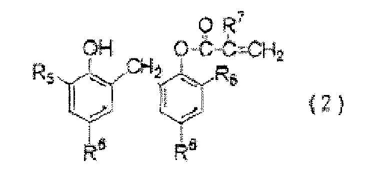
   in the formula (2), R⁵ and R⁶ are each independently an alkyl group having 1 to 18 carbon atoms, an aryl group having 6 to 18 carbon atoms, or an aralkyl group having 7 to 18 carbon atoms; and R⁷ is a hydrogen atom or a methyl group.
[6] Use of the phenol composition of [1] or [2] for stabilizing a thermoplastic polymer during a process.

The phenol compositions of the present invention are capable of further improving the process stability of thermoplastic polymer compositions.

The present invention will be described in detail.

The present invention relates to phenol compositions comprising a compound represented by the formula (1) (hereinafter sometimes referred to as "compound (1)") and a compound represented by the formula (2) (hereinafter sometimes referred to as "compound (2)") in a weight ratio of 1:99 to 99:1.

In the compound (1), R¹ and R² in the formula are each independently an alkyl group having 1 to 8 carbon atoms such as a methyl group, an ethyl group, an n-propyl group, an isopropyl group, an n-butyl group, an isobutyl group, a sec-butyl group, a tert-butyl group, an n-pentyl group, a tert-pentyl group, a 2-ethylhexyl group or a cyclohexyl group; an aryl group having 6 to 18 carbon atoms such as a phenyl group; or an aralkyl group having 7 to 18 carbon atoms such as a benzyl group.

Of these, alkyl groups having a tertiary carbon atom such as a tert-butyl group and a tert-pentyl group are particularly preferable, and a tert-pentyl group is especially preferable.

In the formula (1), R³ is an alkyl group having 1 to 3 carbon atoms such as a methyl group, an ethyl group, an n-propyl group or an isopropyl group, and a methyl group is particularly preferable.

In the formula (1), R⁴ is a hydrogen atom or a methyl group, and a hydrogen atom is particularly preferable.

Specific examples of the compound (1) include
2,4-di-t-butyl-6-[1-(3,5-di-t-butyl-2-hydroxyphenyl)ethyl]phenyl acrylate,
2,4-di-t-amyl-6-[1-(3,5-di-t-amyl-2-hydroxyphenyl)ethyl]phenyl acrylate (compound (1-1)),
2,4-di-t-butyl-6-[1-(3,5-di-t-butyl-2-hydroxyphenyl)ethyl]phenyl metacrylate,
2,4-di-t-amyl-6-[1-(3,5-di-t-amyl-2-hydroxyphenyl)ethyl]phenyl metacrylate,
2,4-di-t-butyl-6-[1-(3,5-di-t-butyl-2-hydroxyphenyl)propyl]phenyl acrylate,
and the like.

Particularly, compound (1-1) is preferable in terms of imparting high process stability to thermoplastic polymers. The compound (1-1) is commercially available as Sumilizer® GS (F) manufactured by Sumitomo Chemical Co., Ltd.

In the compound (2), R⁵ in the formula is an alkyl group having 1 to 18 carbon atoms such as a methyl group, an ethyl group, an n-propyl group, an isopropyl group, an n-butyl group, an isobutyl group, a sec-butyl group, a tert-butyl group, an n-pentyl group, a text-pentyl group, a 2-ethylhexyl group or a cyclohexyl group; an aryl group having 6 to 18 carbon atoms such as a phenyl group; or an aralkyl group having 7 to 18 carbon atoms such as a benzyl group.

Of these, alkyl groups having a tertiary carbon atom such as a tert-butyl group and a tert-pentyl group are particularly preferable, and a tert-butyl group is especially preferable.

In the formula (2), R⁶ is an alkyl group having 1 to 18 carbon atoms such as a methyl group, an ethyl group, an n-propyl group, an isopropyl group, an n-butyl group, an isobutyl group, a sec-butyl group, a tert-butyl group, an n-pentyl group, a tert-pentyl group, a 2-ethylhexyl group or a cyclohexyl group; an aryl group having 6 to 18 carbon atoms such as a phenyl group; or an aralkyl group having 7 to 18 carbon atoms such as a benzyl group. A methyl group is particularly preferable.

R⁵ and R⁶ may be the same or different from each other.

In the formula (2), R⁷ is a hydrogen atom or a methyl group, and a hydrogen atom is particularly preferable.

Specific examples of the compound (2) include
2-t-butyl-6-(3-t-butyl-2-hydroxy-5-methylbenzyl)-4-methylphenyl metacrylate,
2-t-butyl-6-(3-t-butyl-2-hydroxy-5-methylbenzyl)-4-methylphenyl acrylate (compound (2-1)),
2-t-butyl-6-(3-t-butyl-2-hydroxy-5-methylbenzyl)-4-ethylphenyl metacrylate,
2-t-amyl-6-(3-t-amyl-2-hydroxy-5-methylbenzyl)-4-methylphenyl methacrylate,
2-t-amyl-6-(3-t-amyl-2-hydroxy-5-methylbenzyl)-4-methylphenyl acrylate, and the like.

Particularly, compound (2-1) is preferable in terms of imparting high process stability to thermoplastic polymers. The compound (2-1) is commercially available as Sumilizer® GM manufactured by Sumitomo Chemical Co., Ltd.

The composition of the present invention includes the compound (1) and the compound (2) by mixing in a weight ratio of 1:99 to 99:1, preferably from 3:97 to 97:3, particularly from 20:80 to 90:10.

The composition of the present invention may be mixed with a thermoplastic polymer as it is. Further, conventionally known additives may be added to the composition as long as the effects of the present invention are not inhibited, and the mixture can also be used as a process stabilizer of the present invention.

Examples of the additive include antioxidants other than the compound (1) and the compound (2), ultraviolet absorbers, light stabilizers, metal deactivators, nucleating agents, lubricants, antistatic agents, flame retardants, fillers, pigments, inorganic fillers, and the like.

Examples of phenol antioxidants include alkylated monophenols such as 2,6-di-t-butyl-4-methylphenol, 2,4,6-tri-t-butylphenol, 2,6-di-t-butylphenol, 2-t-butyl-4,6-dimethylphenol, 2,6-di-t-butyl-4-ethylphenol, 2,6-di-t-butyl-4-n-butylphenol, 2,6-di-t-butyl-4-isobutylphenol, 2,6-dicyclopentyl-4-methylphenol, 2-(α-methylcyclohexyl)-4,6-dimethylphenol, 2,6-dioctadecyl-4-methylphenol, 2,4,6-tricyclohexylphenol, 2,6-di-t-butyl-4-methoxymethylphenol, 2,6-dinonyl-4-methylphenol, 2,4-dimethyl-6-(1'-methylundecyl-1'-yl)phenol, 2,4-dimethyl-6-(1'-methylheptadecyl-1'-yl)phenol, 2,4-dimethyl-6-(1'-methyltridecyl-1'-yl)phenol, and mixtures thereof;
alkylthiomethylphenols such as 2,4-dioctylthiomethyl-6-t-butylphenol, 2,4-dioctylthiomethyl-6-methylphenol, 2,4-dioctylthiomethyl-6-ethylphenol, 2,6-didodecylthiomethyl-4-nonylphenol, and mixtures thereof;
alkylidene bisphenols and derivatives thereof such as 2,2'-methylenebis(4-methyl-6-t-butylphenol), 2,2'-methylenebis(4-ethyl-6-t-butylphenol), 2,2'-methylenebis[4-methyl-6-(α-methyleyelohexyl)phenol], 2,2'-methylenebis(4-methyl-6-cyclohexylphenol), 2,2'-methylenebis(4-methyl-6-nonylphenol), 2,2'-methylenebis(4,6-di-t-butylphenol), 2,2'-ethylidenebis(4,6-di-t-butylphenol), 2,2'-ethylidenebis(4-isobutyl-6-t-butylphenol), 2,2'-methylenebis[6-(α-methylbenzyl)-4-nonylphenol], 2,2'-methylenebis[6-(α,α,-dimethylbenzyl)-4-nonylphenol], 4,4'-methylenebis(6-t-butyl-2-methylphenol), 4,4'-methylenebis(2,6-di-t-butylphenol), 4,4'-butylidenebis(3-methyl-6-t-butylphenol), 1,1-bis(4-hydroxyphenyl)cyclohexane, 1,1-bis(5-t-butyl-4-hydroxy-2-methylphenyl)butane, 2,6-bis(3-t-butyl-5-methyl-2-hydroxybenzyl)-4-methylphenol, 1,1,3-tris(5-t-butyl-4-hydroxy-2-methylphenyl)butane, 1,1-bis(5-t-butyl-4-hydroxy-2-methylphenyl)-3-n-dodecylmercaptobutane, ethyleneglycolbis[3,3-bis-3'-t-butyl-4'-hydroxyphenyl]butyrate], bis(3-t-butyl-4-hydroxy-5-methylphenyl)dicyclopentadiene, bis[2-(3'-t-butyl-2'-hydroxy-5'-methylbenzyl)-6-t-butyl-4-methylphenyl]terep hthalate, 1,1-bis(3,5-dimethyl-2-hydroxyphenyl)butane, 2,2-bis(3,5-di-t-butyl-4-hydroxyphenyl)propane, 2,2-bis(5-t-butyl-4-hydroxy-2-methylphenyl)-4-n-dodecylmercaptobutane, 1,1,5,5-tetra(5-t-butyl-4-hydroxy-2-methylphenyl)pentane, and mixtures thereof;
acylaminophenol derivatives such as 4-hydroxy-lauric acid anilide,
4-hydroxy-stearic acid anilide, octyl-N-(3,5-di-t-butyl-4-hydroxyphenyl)carbamate, and mixtures thereof;
esters of β-(3,5-di-t-butyl-4-hydroxyphenyl)propionic acid and a monohydric or polyhydric alcohol such as methanol, ethanol, octanol, octadecanol, ethylene glycol, 1,3-propanediol, 1,4-butanediol, 1,6-hexanediol, 1,9-nonanediol, neopentyl glycol, diethylene glycol, thioethylene glycol, spiroglycol, triethylene glycol, pentaerythritol, tris(hydroxyethyl)isocyanurate, N,N'-bis(hydroxyethyl)oxamide, 3-thiaundecanol, 3-thiapentadecanol, trimethylhexanediol, trimethylolpropane,
4-hydroxymethyl-1-phospha-2,6,7-trioxabicyclo[2,2,2]octane and mixtures thereof;
hydroxylated thiodiphenyl ethers such as 2,2'-thiobis(6-t-butylphenol), 2,2'-thiobis(4-methyl-6-t-butylphenol), 2,2'-thiobis(4-octylphenol), 4,4'-thiobis(3-methyl-6-t-butylphenol), 4,4'-thiobis(2-methyl-6-t-butylphenol), 4,4'-thiobis(3,6-di-t-amylphenol), and 4,4'-(2,6-dimethyl-4-hydroxyphenyl)disulfide;
benzyl derivatives including O-benzyl derivatives, N-benzyl derivatives and S-benzyl derivatives such as 3,5,3',5'-tetra-t-butyl-4,4'-dihydroxydibenzyl ether, octadecyl-4-hydroxy-3,5-dimethylbenzyl mercaptoacetate, tris(3,5-di-t-butyl-4-hydroxybenzyl)amine, bis(4-t-butyl-3-hydroxy-2,6-dimethylbenzyl)dithioterephthalate, bis(3,5-di-t-butyl-4-hydroxybenzyl)sulfide, isooctyl-3,5-di-t-butyl-4-hydroxybenzyl mercaptoacetate, and mixtures thereof;
triazine derivatives such as 2,4-bis(n-octylthio)-6-(4-hydroxy-3,5-di-t-butylanilino)-1,3,5-triazine, 2-n-octylthio-4,6-bis(4-hydroxy-3,5-di-t-butylanilino)-1,3,5-triazine, 2-n-octylthio-4,6-bis(4-hydroxy-3,5-di-t-butylphenoxy)-1,3,5-triazine, 2,4,6-tris(3,5-di-t-butyl-4-phenoxy)-1,3,5-triazine, tris(4-t-butyl-3-hydroxy-2,6-dimethylbenzyl)isocyanurate, tris(3,5-di-t-butyl-4-hydroxybenzyl)isocyanurate, 2,4,6-tris(3,5-di-t-butyl-4-hydroxyphenylethyl)-1,3,5-triazine, 2,4,6-tris(3,5-di-t-butyl-4-hydroxyphenylpropyl)-1,3,5-triazine, tris(3,5-dicyclohexyl-4-hydroxybenzyl)isocyanurate, tris[2-(3', 5'-di-t-butyl-4'-hydroxycinnamoyloxy)ethyl]isocyanurate, and mixtures thereof;
hydroxybenzylated malonate derivatives such as dioctadecyl-2,2-bis(3,5-di-t-butyl-2-hydroxybenzyl)malonate, dioctadecyl-2-(3-t-butyl-4-hydroxy-5-methylbenzyl)malonate, didodecylmercaptoethyl-2,2-bis(3,5-di-t-butyl-4-hydroxybenzyl)malonate, bis[4-(1,1,3,3-tetramethylbutyl)phenyl]-2,2-bis(3,5-di-t-butyl-4-hydroxybenz yl)malonate, and mixtures thereof;
aromatic hydroxybenzyl derivatives such as 1,3,5-trimethyl-2,4,6-tris(3,5-di-t-butyl-4-hydroxybenzyl)benzene, 1,4-bis(3,5-di-t-butyl-4-hydroxybenzyl)-2,3,5,6-tetramethylbenzene, 2,4,6-tris(3,5-t-butyl-4-hydroxybenzyl)phenol, and mixtures thereof;
benzyl phosphonate derivatives such as asdimethyl-3,5-di-t-butyl-4-hydroxybenzyl phosphonate, diethyl-3,5-di-t-butyl-4-hydroxybenzyl phosphonate, dioctadecyl-3,5-di-t-butyl-4-hydroxybenzyl phosphonate, dioctadecyl-5-t-butyl-4-hydroxy-3-methylbenzyl phosphonate, calcium salts of 3,5-di-t-butyl-4-hydroxybenzyl phosphonic acid monoester, and mixtures thereof;
esters of β-(5-t-butyl-4-hydroxy-3-methylphenyl)propionic acid and a monohydric or polyhydric alcohol such as methanol, ethanol, octanol, octadecanol, ethylene glycol, 1,3-propanediol, 1,4-butanediol, 1,6-hexanediol, 1,9-nonanediol, neopentyl glycol, diethylene glycol, thioethylene glycol, spiroglycol, triethylene glycol, pentaerythritol, tris(hydroxyethyl)isocyanurate, N,N'-bis(hydroxyethyl)oxamide, 3-thiaundecanol, 3-thiapentadecanol, trimethylhexanediol, trimethylolpropane, 4-hydroxymethyl-1-phospha-2,6,7-trioxabicyclo[2,2,2]octane and mixtures thereof;
esters of β-(3,5-dicyclohexyl-4-hydroxyphenyl)propionic acid and a monohydric or polyhydric alcohol such as methanol, ethanol, octanol, octadecanol, ethylene glycol, 1,3-propanediol, 1,4-butanediol, 1,6-hexanediol, 1,9-nonanediol, neopentyl glycol, diethylene glycol, thioethylene glycol, spiroglycol, triethylene glycol, pentaerythritol, tris(hydroxyethyl)isocyanurate, N,N'-bis(hydroxyethyl)oxamide, 3-thiaundecanol, 3-thiapentadecanol, trimethylhexanediol, trimethylolpropane, 4-hydroxymethyl-1-phospha-2,6,7-trioxabicyclo[2,2,2]octane and mixtures thereof;
esters of 3,5-di-t-butyl-4-hydroxyphenylacetic acid and a monohydric or polyhydric alcohol such as methanol, ethanol, octanol, octadecanol, ethylene glycol, 1,3-propanediol, 1,4-butanediol, 1,6-hexanediol, 1,9-nonanediol, neopentyl glycol, diethylene glycol, thioethylene glycol, spiroglycol, triethylene glycol, pentaerythritol, tris(hydroxyethyl)isocyanurate, N,N'-bis(hydroxyethyl)oxamide, 3-thiaundecanol, 3-thiapentadecanol, trimethylhexanediol, trimethylolpropane, 4-hydroxymethyl-1-phospha-2,6,7-trioxabicyclo[2,2,2]octane and mixtures thereof;
amides of β-(3,5-di-t-butyl-4-hydroxyphenyl)propionic acid such as N,N'-bis[3-(3',5'-di-t-butyl-4'-hydroxyphenyl)propionyl]hydrazine, N,N'-bis[3-(3',5'-di-t-butyl-4'-hydroxyphenyl)propionyl]hexamethylenediami ne,
N.N'-bis[3-(3',5'-di-t-butyl-4'-hydroxyphenyl)propionyl]trimethylenediamine, and mixtures thereof; and
tocopherols such as α-tocopherol, β-tocophexol, γ-tocopherol, δ-tocopherol, and mixtures thereof; and the like.

Examples of hydroquinone and alkylated hydroquinone antioxidants include 2,6-di-t-butyl-4-methoxyphenol, 2,5-di-t-butylhydroquinone, 2,5-di-t-amylhydroquinone, 2,6-diphenyl-4-octadecyloxyphenol, 2,6-di-t-butylhydroquinone, 2,5-di-t-butyl-4-hydroxyanisole, 3,5-di-t'butyl"4"hydroxyphenylstearate, bis(3,5-di-t-butyl-4-hydroxyphenyl)adipate, mixtures threrof, and the like.

Examples of sulfur antioxidants include dilauryl 3,3'-thiodipropionate, tridecyl 3,3'-thiodipropionate, dimyristyl 3,3'-thiodipropionate, distearyl 3,3'-thiodipropionate, laurylstearyl 3,3'-thiodipropionate, neopentanetetrayltetrakis(3-laurylthiopropionate), and the like.

Examples of phosphorus antioxidants include triphenyl phosphite, tris(nonylphenyl) phosphite, tris(2,4-di-t-butylphenyl) phosphite, trilauryl phosphite, trioctadecyl phosphite, distearylpentaerythritol diphosphite, diisodecylpentaerythritol diphosphite, bis(2,4-di-t-butylphenyl) pentaerythritol diphosphite, bis(2,4-di-t-butyl-6-methylphenyl) pentaerythritol diphosphite, bis(2,6-di-t-butyl-4-methylphenyl) pentaerythritol diphosphite, bis(2,4,6-tri-t-butylphenyl) pentaerythritol diphosphite, tristearylsorbitol triphosphite, tetrakis(2,4-di-t-butylphenyl)-4,4'-diphenylene diphosphonite, 2,2'-methylenebis(4,6-di-t-butylphenyl) 2-ethylhexylphosphite, 2,2'-ethylidenebis(4,6-di-t-butylphenyl) fluorophosphite, bis(2,4-di-t-butyl-6-methylphenyl) ethylphosphite, bis(2,4-di-t-butyl-6-methylphenyl) methylphosphite, 2-(2,4,6-tri-t-butylphenyl)-5-ethyl-5-butyl-1,3,2-oxaphosphorinane, 2,2',2"-nitorilo[triethyl-tris(3,3',5,'-tetra-t-butyl-1,1'-biphenyl-2,2'-diyl)phos phite, mixtures thereof, and the like.

Examples of the ultraviolet absorbers include salicylate derivatives such as phenyl salicylate, 4-t-butylphenyl salicylate, 2,4-di-t-butylphenyl-3',5'-di-t-butyl-4'-hydroxybenzoate, 4-t-octylphenyl salicylate, bis(4-t-butylbenzoyl) resorcinol, benzoyl resorcinol, hexadecyl 3',5'-di-t-butyl-4'-hydroxybenzoate, octadecyl 3',5'-di-t-butyl-4'-hydroxybenzoate, 2-methyl-4,6-di-t-butylphenyl 3',5'-di-t-butyl-4'-hydroxybenzoate, and mixtures threrof;
2-hydroxybenzophenone derivatives such as 2,4-dihydroxybenzophenone, 2-hydroxy-4-methoxybenzophenone, 2-hydroxy-4-octoxybenzophenone, 2,2-dihydroxy-4-methoxybenzophenone, bis(5-benzoyl-4-hydroxy-2-methoxyphenyl)methane, 2,2',4,4'-tetrahydroxybenzophenone, and mixtures thereof; and
2-(2'-hydroxyphenyl)benzotriazoles such as 2-(2-hydroxy-5-methylphenyl)benzotriazole, 2-(3',5'-di-t-butyl-2'-hydroxyphenyl)benzotriazole, 2-(5'-t-butyl-2'-hydroxyphenyl)benzotriazole, 2-(2'-hydroxy-5'-t-octylphenyl)benzotriazole, 2-(3-t-butyl-2-hydroxy-5-methylphenyl)-5-chlorobenzotriazole, 2-(3'-s-butyl-2'-hydroxy-5'-t-butylphenyl)benzotriazole, 2-(2'-hydroxy-4'-octyloxyphenyl)benzotriazole, 2-(3',5'-di-t-amyl-2'-hydroxypbenyl)benzotriazole, 2-[2'-hydroxy-3',5'-bis(α,α-dimethylbenzyl)phenyl]-2H-benzotriazole, 2-[(3'-t-butyl-2'-hydroxyphenyl)-5'-(2-octyloxycarbonylethyl)phenyl]-5-chloro benzotriazole, 2-[3'-t-butyl-5'-[2-(2-ethylhexyloxy)caybonylethyl]-2'-hydroxyphenyl]-5-chloro benzotriazole, 2-[3'-t-butyl-2'-hydroxy-5'-(2-methoxycarbonylethyl)phenyl]-5-chlorobenzotri azole, 2-[3'-t-butyl-2'-hydroxy-5'-(2-methoxycarbonylethyl)phenyl]benzotriazole, 2-[3'-t-butyl-2'-hydroxy-5-(2-octyloxycarbonylethyl)phenyl]benzotriazole, 2-[3'-t-butyl-2'-hydroxy-5'-[2-(2-ethylhexyloxy)carbonylethyl]phenyl]benzotri azole, 2-[2-hydroxy-3-(3,4,5,6-tetrahydrophthalimidemethyl)-5-methylphenyl]benz otriazole, 2-(3,5-di-t-butyl-2-hydroxyphenyl)-5-chlorobenzotriazole, mixtures of 2-(3'-dodecyl-2'-hydroxy-5'-methylphenyl)benzotriazole and 2-[3'-t-butyl-2'-hydroxy-5'-(2-isooetyloxycarbonylethyl)phenyl]benzotriazole, 2,2'-methylenebis[6-(2H-benzotriazol-2-yl)-4-(1,1,3,3-tetramethylbutyl)phen ol, 2,2'-methylenebis[4-t-butyl-6-(2H-benzotriazol-2-yl)phenol], condensation products of poly(3 to 11)(ethylene glycol) and 2-[3'-t-butyl-2'-hydroxy-5'-(2-methoxycarbonylethyl)phenyl]benzotriazole, condensation products of poly(3 to 11)(ethylene glycol) and methyl 3-[3-(2H-benzotriazol-2-yl)-5-t-butyl-4-hydroxyphenyl]propionate, 2-ethylhexyl 3-[3-t-butyl-5-(5-chloro-2H-benzotriazol-2-yl)-4-hydroxyphenyl]propionate, octyl 3-[3-t-butyl-5-(5-chloro-2H-benzotriazol-2-yl)-4-hydroxyphenyl]propionate, methyl 3-[3-t-butyl-5-(5-chloro-2H-benzotriazol-2-yl)-4-hydroxyphenyl]propionate, 3-[3-t-butyl-5-(5-chloro-2H-benzotriazol-2-yl)-4-hydroxyphenyl]propionic acid, and mixtures thereof; and the like.

Examples of the light stabilizer include bis(2,2,6,6-tetramethyl-4-piperidyl)sebacate, bis(2,2,6,6-tetramethyl-4-piperidyl)succinate, bis(1,2,2,6,6-pentamethyl-4-piperidyl)sebacate, bis(N-octoxy-2,2,6,6-tetramethyl-4-piperidyl) sebacate, bis(N-benzyloxy-2,2,6,6-tetramethyl-4-piperidyl) sebacate, bis(N-cyclohexyloxy-2,2,6,6-tetramethyl-4-piperidyl) sebacate, bis(1,2,2,6,6-pentamethyl-4-piperidyl) 2-(3,5-di-t-butyl-4-hydroxybenzyl)-2-butylmalonate, bis(1-acroyl-2,2,6,6-tetramethyl-4-piperidyl) 2,2-bis(8,5-di-t-butyl-4-hydroxybenzyl)-2-butylmalonate, bis(1,2,2,6,6-pentamethyl-4-piperidyl decanedioate, 2,2,6,6-tetramethyl-4-piperidyl methacrylate, 4-[3-(3,5-di-t-butyl-4-hydroxyphenyl)propionyloxyl-1-[2-(3-(3,5-di-t-butyl-4-h ydroxyphenyl)propionyloxy)ethyl]-2,2,6,6-tetramethyl piperidine, 2-methyl-2-(2,2,6,6-tetramethyl-4-piperidvl)amino-N-(2,2,6,6-tetramethyl-4-piperidyl) propionamide, tetrakis(2,2,6,6-tetramethyl-4-piperidyl) 1,2,3,4-butanetetracarboxylate, tetrakis(1,2,2,6,6-pentamethyl-4-piperidyl) 1,2.3,4-butaneteracarboxylate, mixed esters of 1,2,3,4-butanetetracarboxylic acid with 1,2,2,6,6-pentamethyl-4-piperidinol and 1-tridecanol;
hindered amine light stabilizers such as mixed esters of 1,2,3,4-butanetetracarboxylic acid with 2,2,6,6-tetramethyl-4-piperidinol and 1-tridecanol, mixed esters of 1,2,3,4-butanetetracarboxylic acid with 1,2,2,6,6-pentamethyl-4-piperidinol and 3,9-bis(2-hydroxy-1,1-dimethylethyl)-2,4,8,10-tetraoxaspiro[5.5]undecane, mixed esters of 1,2,3,4-butanetetracarboxylic acid with 2,2,6,6-tetramethyl-4-piperidinol and 3,9-bis(2-hydroxy-1,1-dimethylethyl)-2,4,8,10-tetraoxaspiro[5.5]undecane, polycondensation products of dimethyl succinate with 1-(2-hydroxyethyl)-4-hydroxy-2,2,6,6-tetramethyl piperidine, poly[(6-morpholino-1,3,5-triazine-2,4-diyl)((2,2,6,6-tetramethyl-4-piperidyl)i mino)hexamethylene((2,2,6,6-tetramethyl-4-piperidyl)imino)], poly(6-(1,1,3,3-tetramethylbutyl)imino-1,3,5-triazine-2,4-diyl((2,2,6,6-tetram ethyl-4-piperidyl)imino)hexamethylene((2,2,6,6-tetramethyl-4-piperidyl)imi no)),
polycondensation products of N,N'-bis(2,2,6,6-tetramethyl-4-piperidyl)hexamethylenediamine with 1,2-dibromoethane, N,N',4,7-tetrakis[4,6-bis(N-butyl-N-(2,2,6,6-tetramethyl-4-piperidyl)amino)-1,3,5-triazine-2-yl]-4,7-diazadecane-1,10-diamine, N,N',4-tris[4,6-bis(N-butyl-N-(2,2,6,6-tetramethyl-4-piperidyl)amino)-1,3,5-t riazine-2-yl]-4,7-diazadecanel, 10-diamine, N,N',4,7-tetrakis[4,6-bis(N-butyl-N-(1,2,2,6,6-pentamethyl-4-piperidyl)amin o)-1,3,5-triazine-2-yl]-4,7-diazadecane-1,10-diamine, N,N',4-tris[4,6-bis(N-butyl-N-(1,2,2,6,6-pentamethyl-4-pipendyl)amino)-1,3, 5-triazine-2-yl]-4,7-diazadecane-1,10-diamine, and mixtures thereof;
acrylic light stabilizers such as ethyl α-cyano-β,β-diphenylacrylate, isooctyl α-cyano-β,β-diphenylacrylate, methyl α-carbomethoxycinnamate, methyl α-cyano-β-methyl-p-methoxycinnamate, butyl α-cyano-β-methyl-p-methoxycinnamate, methyl α-carbomethoxy-p-methoxycinnamate, N-(β-carbomethoxy-β-cyanovinyl)-2-methylindoline, and mixtures thereof;
nickel light stabilizers such as nickel complexes of 2,2'-thiobis-[4-(1,1,3,3-tetramethylbutyl)phenol], nickel dibutyldithiocarbamate, nickel salts of monoalkyl ester, nickel complexes of ketoxime, and mixtures thereof;
oxamide light stabilizers such as 4,4'-dioctyloxyoxanilide, 2,2'-diethoxyoxanilide, 2,2'-dioctyloxy-5,5'-di-t-butyl anilide, 2,2'-didodecyloxy-5,5'-di-t-butyl anilide, 2-ethoxy-2'-ethyl oxanilide, N,N'-bis(3-dimethylaminopropyl)oxamide, 2-ethoxy-5-t-butyl-2'-ethoxyanilide, 2-ethoxy-5,4'-di-t-butyl-2'-ethyl oxanilide, and mixtures thereof; and
2-(2-hydroxyphenyl)-1,3,5-triazine light stabilizers such as 2,4,6-tris(2-hydroxy-4-octyloxyphenyl)-1,3,5-triazine, 2-(2-hydroxy-4-octyloxyphenyl)-4,6-bis(2,4dimethylphenyl)-1,3,5-triazine, 2-[2,4-dihydroxyphenyl-4,6-bis(2,4-dimethylphenyl)-1,3,5-triazine, 2,4-bis(2-hydroxy-4-propyloxyphenyl)-6-(2,4-dimethylphenyl)-1,3,5-triazine, 2-(2-hydroxy-4-octyloxyphenyl)-4,6-bis(4-methylphenyl)-1,3,5-triazine, 2-(2-hydroxy-4-dodecyloxyplaenyl)-4,6-bis(2,4dimethylphenyl)-1,3,5-triazine, 2-[2-hydroxy-4-(2-hydroxy-3-butyloxypropoxy)phenyl]-4,6-bis(2,4-dimethylp henyl)-1,3,5-triazine, 2-[2-hydraxy-4-(2-hydroxy-3-octylaxypropoxy)phenyl]-4,6-bis(2,4dimethylphe nyl)-1,3,5-triazine, and mixtures thereof; and the like.

In addition to the stabilizes as listed above, examples thereof also include hydroxyl amines such as N,N-dibenzylhydroxyamine, N,N-diethylhydroxyamine, N,N-dioctylhydroxyamine, N,N-dilaurylhydroxyamine, N,N-ditetradecylhydroxyamine, N,N-dihexadecylhydroxyamine, N,N-dioctadecylhydroxyamine, N-hexadecyl-N-octadecylhydroxyamine, N-heptadecyl-N-octadecylhydroxyamine, and mixtures thereof; and the like.

Examples of the lubricant include aliphatic hydrocarbons such as paraffin and waxes, higher fatty acids having 8 to 22 carbon atoms, metal (Al, Ca, Mg, Zn) salts of higher fatty acid having 8 to 22 carbon atoms, aliphatic alcohols having 8 to 22 carbon atoms, polyglycols, esters of higher fatty acid having 4 to 22 carbon atoms and aliphatic monohydric alcohol having 4 to 18 carbon atoms, higher fatty amides having 8 to 22 carbon atoms, silicone oils, rosin derivatives, and the like. The phenol antioxidants other than the compounds (1) and (2), the phosphorus antioxidants, the sulfur antioxidants, the ultraviolet absorbers, and the hindered amine light stabilizers are preferably used, among of these additives other than the compounds (1) and (2).

Examples of the particularly preferable phenol antioxidant other than the compounds (1) and (2) include 2,6-di-t-butyl-4-methylphenol, 2,4,6-tri-t-butylphenol, 2,4-dioctylthiomethyl-6-methylphenol, 2,2'-thiobis(6-t-butylphenol), 4,4'-thiobis(3-methyl-6-t-butylphenol), 2,2'-methylenebis(4-methyl-6-t-butylphenol), 2,2'-methylenebis(4-ethyl-6-t-butylphenol), 2,2'-methylenebis[4-methyl-6-(α-methylcyclohexyl)phenol], 2,2'-methylenebis(4-methyl-6-cyclohexylphenol), 2,2'-methylenebis(4,6-di-t-butylphenol), 2,2'-ethylidenebis(4,6,-di-t-butylphenol), 4,4'-methylenebis(6-t-butyl-2-methylphenol), 4,4'-methylenebis(2,6-di-t-butylphenol), 4,4'-butylidenebis(3-methyl-6-t-butylphenol), 1,1-bis(4-hydraxyphenyl)cyclohexane, 1,1-bis(5-t-butyl-4-hydroxy-2-methylphenyl)butane, 1,1,3-tris(5-t-butyl-4-hydroxy-2-methylphenyl)butane, ethyleneglycolbis [3,3-bis-3'-t-butyl-4'-hydroxyphenyl]butyrate; and
2,4,6-tris(3,5-di-t-butyl-4-phenoxy)-1,3,5-triazine, tris(4-t-butyl-3-hydroxy-2,6-dimethylbenzyl)isocyanurate, bis(3,5-di-t-butyl-4-hydroxybenzyl)isocyanurate, tris[2-(3',5'-di-t-butyl-4'-hydroxycinnamoyloxy)ethyl]isacyanurate, diethyl-3,5-di-t-butyl-4-hydroxybenzyl phosphonate, di-n-octadecyl-3,5-di-t-butyl-4-hydroxybenzyl phosphonate, calcium salts of 3,5-di-t-butyl-4-hydroxybenzyl phosphonic acid monoester, n-octadecyl 3-(3,5-di-t-butyl-4-hydroxyphenyl)propionate, neopentanetetrayltetrakis(3,5-di-t-butyl-4-hydroxycinnamate), thiodiethylenebis(3,5-di-t-butyl-4-hydroxycinnamate), 1,3,5-trimethyl-2,4,6-tris(3,5-di-t-butyl-4-hydroxybenzyl)benzene, 3,6-dioxaoctamethylenebis(3,5-di-t-butyl-4-hydroxycinnamate), hexamethylenebis(3,5-di-t-butyl-4-hydroxycinnamate), triethyleneglycolbis(5-t-butyl-4-hydroxy-3-methylcinnamate), 3,9-bis[2-(3-(3-t-butyl-4-hydroxy-5-methylphenyl)propionyloxy)-1, 1-dimethyl ethyl]-2,4,8,10-tetraoxaspero[3.5]undecane, N,N'-bis[3-(3',5'-di-t-butyl-4-hydroxyphenyl)propionyl]hydrazine, N,N'-bis[3-(3',5'-di-t-butyl-4'-hydroxyphenyl)propionyl]hexamethylenediami ne, and the like. They may be used alone or in combination.

Examples of the particularly preferable phosphrous antioxidant include tris(nonylphenyl) phosphite, tris(2,4-di-t-butylphenyl) phosphite, distearylpentaerythritol diphosphite, bis(2,4-di-t-butylphenyl) pentaerythritol diphosphite, bis(2,4-di-t-butyl-6-methylphenyl) pentaerythritol diphosphite, bis(2,6-di-t-butyl-4-methylphenyl) pentaerythritol diphosphite, tetrakis(2,4-di-t-butylphenyl)-4,4'-diphenylene diphosphonite, 2,2'-methylenebis(4,6-di-t-butylphenyl) 2 -ethylhexylphosphite, 2,2'-ethylidenebis(4,6-di-t-butylphenyl)fluorophosphite, bis(2,4-di-t-butyl-6-mothylphenyl) ethyl phosphite, 2-(2,4,6-tri-t-butylphenyl)-5-ethyl-5-butyl-1,3,2-oxaphosphorinane, 2,2',2"-nitrilo[triethyl-tris(3,3',5,5'-tetra-t-butyl-1,1'-biphenyl-2,2'-diyl) phosphite, and the like. They may be used alone or in combination.

Examples of the preferable ultraviolet absorber include phenyl salicylate, 4-t-butylphenyl salicylate, 2,4-di-t-butylphenyl 3',5'-di-t-butyl-4'-hydroxybenzoate, 4-t-octylphenyl salicylate, 2,4-dihydroxybenzophenone, 2-hydroxy-4-methoxybenzophenone, 2-hydroxy-4-octoxybenzophenone, 2,2'-dihydroxy-4-methoxybenzophenone, bis(5-benzoyl-4-hydroxy-2-methoxyphenyl)methane, 2,2',4,4'-tetrahydroxybenzophenone, 2-(2-hydroxy-5-methylphenyl)benzotriazole, 2-(3',5'-di-t-butyl-2'-hydroxyphenyl)benzotriazole, 2-(5'-t-butyl-2'-hydroxyphenyl)benzotriazole, 2-(2'-hydroxy-5'-t-octylphenyl)benzotriazole, 2-(3-t-butyl-2-hydroxy-5-methylphenyl)-5-chlorobenzotriazole, 2-(3'-s-butyl-2'-hydroxy-5'-t-butylphenyl)benzotriazole, 2-(2'-hydroxy-4'-octyloxyphenyl)benzotriazole, 2-(3',5'-di-t-amyl-2'-hydroxyphenyl)benzotriazole, 2-[2'-hydroxy-3',5'-bis(α,α,-dimethylbenzyl)phenyl]-2H-benzotriazole, and the like. They may be used alone or in combination.

Examples of the preferable light stabilizer include bis(2,2,6,6-tetramethyl-4-piperidyl) sebacate, bis(1,2,2,6,6-pentamethyl-4-piperidyl) sebacate, bis(N-octoxy-2,2,6,6-tetramethyl-4-piperidyl) sebacate, bis(N-benzyloxy-2,2,6,6-tetramethyl-4-piperidyl) sebacate, bis(N-cyclohexyloxy-2,2,6,6-tetramethyl-4-piperidyl) sebacate, bis(1,2,2,6,6-pentamethyl-4-piperidyl) 2-(3,5-di-t-butyl-4-hydroxybenzyl)-2-butylxnalonate, bis(1-acroyl-2,2,6,6-tetramethyl-4-piperldyl) 2,2-bis(3,5-di-t-butyl-4-hydroxybenzyl)-2-butylmalonate, bis(2,2,6,6-tetramethyl-4-piperidyl) succinate, 2,2,6,6-tetramethyl-4-piperidyl methacrylate, 4-[3-(3,5-di-t-butyl-4-hydroxyphenyl)propionyloxy]-1-[2-(3-(3,5-di-t-butyl-4-h ydroxyphenyl)propionyloxy)ethyl]-2,2,6,6-tetramethylpiperidine, 2-methyl-2-(2,2,6,6-tetramethyl-4-piperidyl)amino-N-(2,2,6,6-tetramethyl-4-piperidyl)propionamide, tetrakis(2,2,6,6-tetramethyl-4-piperidyl) 1,2,3,4-butanetetracarboxylate; and
tetrakis(1,2,6,6-pentamethyl-4-piperidyl) 1,2,3,4-butanetetracarboxylate, mixed esters of 1,2,3,4-butanetetracarboxylic acid with 1,2,2,6,6-pentamethyl-4-piperidinol and 1-tridecanol, mixted esters of 1,2,3,4-butanetetracarboxylic acid with 2,2,6,6-tetramethyl-4-piperidinol and 1-tridecanol, mixed esters of 1,2,3,4-butanetetracarboxylic acid with 1,2,2,6,6-pentamethyl-4-piperidinol and 3,9-bis(2-hydroxy-1,1-dimethylethyl)-2,4,8,10-tetraoxaspiro[5,5]undecane, mixed esters of 1,2,3,4-butanetetracarboxylic acid with 2,2,6,6-tetramethyl-4-piperidinol and 3,9-bis(2-hydroxy-1,1-dimethylethyl)-2,4,8,10-tetraoxaspiro[5.5]undecane, polycondensation products of dimethyl succinate with 1-(2-hydroxyethyl)-4-hydroxy-2,2,6,6-tetramethylpiperidine, poly[6-morpholino-1,3,5-triazine-2,4-diyl)((2,2,6,6-tetramethyl-4-piperidyl)i mino)hexamethylene((2,2,6,6-tetramethyl-4-piperidyl)imino)], poly[(6-(1,1;3,3-tetramethylbutyl)-1,3,5-triazine-2,4-diyl)((2,2,6,6-tetramethy 1-4-piperidyl)imino)hexamethylene((2,2,6,6-tetramethyl-4-piperidyl)imino)], and the like. They may be used alone or in combination.

As a process for producing the phenol composition of the present invention, for example, a process in which the compound (1), the compound (2) and, if necessary, the additives as listed above are mixed in a mixer such as a Henschel mixer, a super mixer or a high speed mixer; a process in which a mixture of the compound (1), the compound (2), and the additives is extruded; a process in which a mixture of the compound (1), the compound (2) and the additives is further stirred and granulated; and the like are exemplified.

The thermoplastic polymer composition of the present invention is a thermoplastic polymer composition comprising a thermoplastic polymer, the compound (1) and the compound (2), wherein a weight ratio of the compound (1) and the compound (2) is from 1:99 to 99:1. The composition includes the compound (1) and the compound (2) in a total amount of preferably 0.01 to 5 parts by weight, particularly preferably 0.02 to 2 parts by weight, especially preferably 0.02 to 1 part by weight based on 100 parts by weight of the thermoplastic polymer.

Examples of the thermoplastic polymer include polypropylene resins such as ethylene-propylene copolymers, polyethylene resins (high density polyethylene (HD-PE), low density polyethylene (LD-PE), linear low density polyethylene (LLDPE), and the like), methylpentene polymers, ethylene-ethyl acrylate copolymers, ethylene-vinyl acetate copolymers, polystyrenes (polystyrene such as poly(p-methyl styrene) or poly(α-methyl styrene), acrylnitrile-styrene copolymers, acrylonitrile-butadiene-styrene copolymers, special acrylic rubber-acrylonitrile-styrene copolymers, acrylonitrile-chlorinated polyethylene-styrene copolymers, styrene-butadiene copolymers, and the like), chlorinated polyethylene, polychloroprene, chlorinated rubber, polyvinyl chloride, polyvinylidene chloride, methacrylic resins, ethylene-vinyl alcohol copolymers, fluorine resins, polyacetal, grafted polyphenylene ether resins, polyphenylene sulfide resins, polyurethane, polyamide, polyester resins (for example, polyethylene terephthalate, polybutylene terephthalate, and the like), polycarbonate, polyacrylate, polysulfone, polyether ether ketone, polyether sulfone, aromatic polyester resins, dially phthalate prepolymers, silicone resins, 1,2-polybutadiene, polyisoprene, butadiene/acrylonitrile copolymers, ethylene-methyl methacrylate copolymers, and the like. Of these, from the viewpoint of good molding processability, polyethylene resins, polypropylene resins and polystyrenes are preferable, and polypropylene resins, acrylonitrile -butadiene- styrene copolymers, and styrene-butadiene copolymers are especially preferable.

The polypropylene resin means polyolefins containing structure units derived from propylene, specific examples thereof include crystalline propylene homopolymers, propylene ethylene randam copolymers, propylene-α-olefin randam copolymers, propylene-ethylene-α-olefin copolymers, polypropylene block copolymers composed of a propylene homopolymer component or a copolymer component mainly containing propylene, and a copolymer component of propylene and ethylene and/or α-olefin, and the like.

In the present invention, when the polypropylene resin is used as the thermoplastic polymer, it may be used alone or in combination.

The α-olefin usually has 4 to 12 carbon atoms, and examples thereof include 1-butene, 1-pentene, 1-hexene, 4-methyl-1-pentene, 1-octene, 1-decene, and the like. Of these, 1-butene, 1-hexene, and 1-octene are more preferable.

Examples of the propylene-ethylene-α-lefin copolymer include propylene-ethylene-1-butene copolymers, propylene-ethylene-1-hexene copolymers, propylene-ethylene-1-octene copolymers, and the like.

Examples of the copolymer component including mainly propylene in the polypropylene blocked copolymer composed of a propylene homopolymer component or a copolymer component containing mainly propylene, and a copolymer component of propylene and ethylene and/or α-olefin include propylene-ethylene copolymer components, prapylene-1-butene copolymer components, propylene-1-hexene copolymer components, and the like; and examples of the copolymer component of propylene and ethylene and/or α-olefin include propylene-ethylene copolymer components, propylene-ethylene-1-butene copolymer components, propylene-ethylene-1-hexene copolymer components, propylene-ethylene-1-octene copolymer components, propylene-1-butene copolymer components, propylene-1-hexene copolymer components, propylene-1-octene copolymer components, and the like. The content of ethylene and/or α-olefin having 4 to 12 carbon atoms in the copolymer component of propylene and ethylene and/or α-olefin is usually from 0.01 to 20% by weight.

Examples of the polypropylene block copolymer composed of a propylene homopolymer component or a copolymer component mainly containing propylene and a copolymer component of propylene and ethylene and/or α-olefin include propylene-ethylene block copolymers, (propylene)-(propylene-ethylene) block copolymers, (propylene)-(propylene-ethylene-1-butene) block copolymers, (propylene)-(propylene-ethylene-1-hexene) block copolymers, (propylene)-(propylene-1-butene) block copolymers, (propylene)-(propylene-1-hexene) block copolymers, (propylene-ethylene)-(propylene-ethylene-1-butene) block copolymers, (propylene-ethylene)-(propylene-ethylene-1-hexene) block copolymers, (propylene-ethylene)-(propylene-1-butene) block copolymers, (propylene-ethylene)-(propylene-1-hexene) block copolymers, (propylene-1-butene)-(propylene-ethylene) block copolymers, (propylene-1-butene)-(propylene-ethylene-1-butene) block copolymers, (propylene-1-butene)-(propylene-ethylene-1-hexene) block copolymers, (propylene-1-butene)-(propylene-1-butene) block copolymers, (propylene-1-butene)-(propylene-1-hexene) block copolymers, and the like.

In the present invention, when the polypropylene resin is used as the thermoplastic polymer, the crystalline propylene homopolymers, and the polypropylene block copolymers composed of a propylene homopolymer component or a copolymer component mainly containing propylene and a copolymer component of propylene and ethylene and/or α-olefin having 4 to 12 carbon atoms are preferable, and the polypropylene block copolymer composed of a propylene homopolymer component or a copolymer component mainly containing propylene and a copolymer component of propylene and ethylene and/or α-olefin having 4 to 12 carbon atoms are more preferable.

The process for producing a thermoplastic polymer composition is a process comprising the step of mixing the compound (1) and the compound (2). Specifically, for example, there is a process comprising the step of separately mixing the compound (1), the compound (2) and, if necessary, the additives with a thermoplastic polymer, upon kneading the thermoplastic polymer; or a process comprising the steps of mixing of the compound (1), the compound (2) and, if necissary, the additives to produce a phenol composition, and mixing the phenol composition obtained in the previous step with a thermoplastic polymer, and the like. The latter process is preferable from the viewpoint of the dispersibility of the compound (1) and the compound (2) in the thermoplastic polymer.

The step of mixing a phenol composition with a thermoplastic polymer will be described in more detail. That is, for example, a process in which the thermoplastic polymer and the phenol composition as described above are dry-blended, and the mixture is melt-kneaded in a single-screw or twin-screw extruder and then extruded to give pellets of the thermoplastic polymer; a process in which the phenol composition is dissolved in a solvent such as cyclohexane to give a solution, and the solution is added to a polymer solution after the completion of polymerizing a thermoplastic polymer, and the solvent is removed from the mixture, and the like are exemplified.

The thus obtained thermoplastic polymer compositions are preferably used for, for example, packaging containers for food, convenience goods, electronic and electric components, component materials of transportation equipments such as automobiles, and the like.

### (Examples)

The present invention will be described in more detail by means of examples and comparative examples, but the invention is not limited thereto. Unless otherwise indicated, parts and % are by weight.

### Example 1

### (Production Example of Phenol Composition 1)

In a mortar, 0.125 parts of 2,4-di-t-amyl-6-[1-(3,5-di-t-amyl-2-hydroxyphenyl)ethyl]phenyl acrylate (Sumilizer® GS(F) manufactured by Sumitomo Chemical Co., Ltd.), that is compound (1-1), as compound (1), and 0.375 parts of 2-t-butyl-6-(3-t-butyl-2-hydroxy-5-methylbenzyl)-4-methylphenyl acrylate (Sumilizer® GM manufactured by Sumitomo Chemical Co., Ltd.), that is compound (2-1), as compound (2) were mixed to produce a phenol composition.

### (Production Example of Thermoplastic Polymer Composition 1)

As a thermoplastic polymer, a styrene-butadiene copolymer (hereinafter sometimes referred to as "SBS." MI (at 200°C and under a load of 5.0 kg): 5 g/10 minutes, Asaflex 830 manufactured by Asahi Kasei Chemicals Corporation) was used, and 100 parts thereof was dry-blended with 0.5 parts of the phenol composition obtained in (Production Example of Phenol Composition 1), and then the mixture was kneaded in a Labo Plastmill (manufactured by Toyo Seiki Seisaku-sho, Ltd. Type: 4C 150-0 1) at 250°C under nitrogen atmosphere at 30 rpm for 5 minutes to produce a thermoplastic polymer composition.

### (Process Stability Test)

The thermoplastic polymer composition obtained in (Production Example of Thermoplastic Polymer Composition 1) was molded into a sheet-shaped form having a thickness of 1 mm through a pressing machine at 230°C. The sheet was cut into chips, and a melt flow rate (MFR) value of the chip was measured at 230°C under a load of 2.16 kg using a melt indexer (L217-E14011 manufactured by TechnoSeven Co., Ltd.) in accordance with JIS K 7210. When SBS is used as the thermoplastic polymer, it means that the higher the MFR value measured, the better the process stability.

The test results are shown in Table 1.

**[Table 1]**

| | SBS (part) | compound (1-1) (part) | compound (2-1) (part) | process stability | |
|---|---|---|---|---|---|
| | | | | MFR value (at 230°C, under 2.16 kg) | relative ratio^{*1} |
| Example 1 | 100 | 0.125 | 0.375 | 7.72 | 121 |
| Example 2 | 100 | 0.450 | 0.050 | 7.02 | 110 |
| Example 3 | 100 | 0.485 | 0.015 | 6.76 | 106 |
| Comparative Example 1 | 100 | 0.500 | 0.000 | 6.37 | 100 |
| Comparative Example 2 | 100 | 0.000 | 0.500 | 6.21 | 98 |
| Comparative Example 3 | 100 | 0.000 | 0.000 | 4.21 | 66 |

| | | | | | |
|---|---|---|---|---|---|
| *1: Relatives ratios of MFR values when the MFR value of Comparative Example 1 is assumed to be 100. | | | | | |

### Examples 2 and 3, and Comparative Example 1 to 3

The same procedure of Example 1 was carried out except that compound (1-1) and compound (1-2) were used in amounts shown in Table 1 as a phenol composition. The results were summarized in Table 1.

The compositions of Examples 1 to 3 all had larger MFR values than those of the thermoplastic polymer composition comprising compound (I-1) alone (Comparative Example 1), the thermoplastic polymer composition comprising compound (2-1) alone (Comparative Example 2), and the composition of the thermoplastic polymer alone (Comparative Example 3), and therefore it can be seen that they have good process stability.

### Example 4

### (Production Example of Phenol Composition 2)

The same procedure of Example 1 was carried out except that compound (1-1) and compound (1-2) were used in amounts shown in Table 2 to give a phenol composition.

### (Production Example of Thermoplastic Polymer Composition 2)

A propylene ethylene block copolymer (hereinafter sometimes referred to as "PP·EP". MI (at 230°C, under a load of 2.16 kg): 9 to 10 g/10 minutes, manufactured by Sumitomo Chemical Co., Ltd.) was used as a thermoplastic polymer, and 100 parts thereof was dry-blended with 0.5 parts of the phenol composition obtained in (Production Example of Phenol Composition 2), and then the mixture was kneaded at 230°C using a twin-screw extruder with a 30 mm diameter (NAS type 30 mm φ twin-screw vent extruder, L/D:28, manufactured by Nakatani Machinery Co., Ltd.) at a screw speed of 80 rpm to give pellets of a thermoplastic polymer composition.

### (Process Stability Test)

The thermoplastic polymer composition obtained in (Production Example of Thermoplastic Polymer Composition 2) was molded into a sheet-shaped form having a thickness of 1 mm through a pressing machine at 230°C. The sheet was cut into chips, and a melt flow rate (MFR) value of the chip was measured at 230°C under a load of 2.16 kg using a melt indexer (L217-E14011 manufactured by TechnoSeven Co., Ltd.) in accordance with JIS K 7210. When PP·EP is used as the thermoplastic polymer, it means that the smaller the MFR value measured, the better the process stability.

The resutls are shown in Table 2.

**[Table 2]**

| | PP·EP (part) | compound (1-1) (part) | compound (2-1) (part) | process stability | |
|---|---|---|---|---|---|
| | | | | MFR value (at 230°C, under 2.16 kg) | relative ratio^{*1} |
| Example 4 | 100 | 0.125 | 0.375 | 10.8 | 89 |
| Example 5 | 100 | 0.450 | 0.050 | 10.7 | 88 |
| Comparative Example 4 | 100 | 0.000 | 0.000 | 12.2 | 100 |

| | | | | | |
|---|---|---|---|---|---|
| *1: Relatives ratios of MFR values when the MFR value of Comparative Example 4 is assumed to be 100. | | | | | |

### Example 5 and Comparative Example 4

The same procedure of Example 4 was carried out except that compound (1-1) and compound (1-2) were used in amounts shown in Table 2 as a phenol composition. The results were summarized in Table 2.

The compositions of Examples 4 and 5 all had smaller MFR values than that of the composition of the thermoplastic polymer alone (Comparative Example 4), and therefore it can be seen that they have good process stability.

The phenol compositions of the present invention are capable of further improving the process stability of the thermoplastic polymer compositions.

## Claims

1. A phenol composition comprising:
a compound represented by the formula (1): 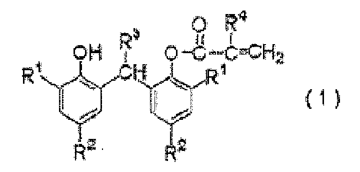
in the formula (1), R¹ and R² are each independently an alkyl group having 1 to 8 carbon atoms, an aryl group having 6 to 18 carbon atoms, or an aralkyl group having 7 to 18 carbon atoms; R³ is an alkyl group having 1 to 3 carbon atoms; and R⁴ is a hydrogen atom or a methyl group, and a compound represented by the formula (2): 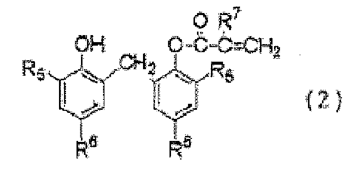
in the formula (2), R⁵ and R⁶ are each independently an alkyl group having 1 to 18 carbon atoms, an aryl group having 6 to 18 carbon atoms, or an aralkyl group having 7 to 18 carbon atoms; and R⁷ is a hydrogen atom or a methyl group, in a weight ratio of 1:99 to 99:1.

2. The phenol composition of Claim 1, wherein the compound represented by the formula (1) is
2,4-di-t-amyl-6-[1-(3,5-di-t-amyl-2-hydroxyphenyl)ethyl]phenyl acrylate, and the compound represented by the formula (2) is 2-t-butyl-6-(3-t-butyl-2-hydroxy-5-methylbenzyl)-4-methylphenyl acrylate.

3. A thermoplastic polymer composition comprising:
a thermoplastic polymer, a compound represented by the formula (1): 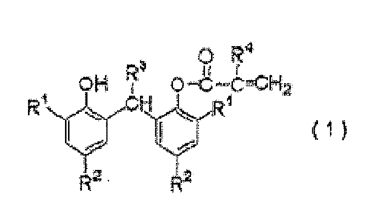
in the formula (1), R¹ and R² are each independently an alkyl group having 1 to 8 carbon atoms, an aryl group having 6 to 18 carbon atoms, or an aralkyl group having 7 to 18 carbon atoms; R³ is an alkyl group having 1 to 3 carbon atoms; and R⁴ is a hydrogen atom or a methyl group, and a compound represented by the formula (2): 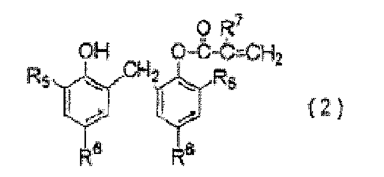
in the formula (2), R⁵ and R⁶ are each independently an alkyl group having 1 to 18 carbon atoms, an aryl group having 6 to 18 carbon atoms, or an aralkyl group having 7 to 18 carbon atoms; and R⁷ is a hydrogen atom or a methyl group, wherein
a weight ratio of the compound represented by the formula (1) to the compound represented by the formula (2) in the composition is from 1:99 to 99:1.

4. The thermoplastic polymer composition of Claim 3, wherein the total contents of the compound represented by the formula (1) and the compound represented by the formula (2) is from 0.01 to 5 parts by weight based on 100 parts by weight of the thermoplastic polymer.

5. A process for producing a thermoplastic polymer composition comprising the step of:
mixing a thermoplastic polymer with a compound represented by the formula (1): 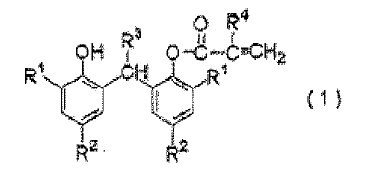
in the formula (1), R¹ and R² are each independently an alkyl group having 1 to 8 carbon atoms, an aryl group having 6 to 18 carbon atoms, or an aralkyl group having 7 to 18 carbon atoms; R³ is an alkyl group having 1 to 3 carbon atoms; and R⁴ is a hydrogen atom or a methyl group and a compound represented by the formula (2): 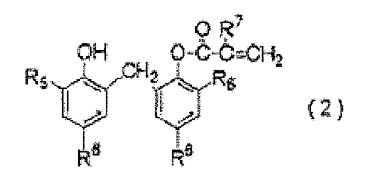
in the formula (2), R⁵ and R⁶ are each independently an alkyl group having 1 to 18 carbon atoms, an aryl group having 6 to 18 carbon atoms, or an aralkyl group having 7 to 18 carbon atoms; and R⁷ is a hydrogen atom or a methyl group.

6. Use of the phenol composition of Claim 1 or 2 for stabilizing a thermoplastic polymer during a process.
